# EUROPÄISCHE PATENTSCHRIFT

(11) **EP 1 067 192 B1**
(45) Veröffentlichungstag und Bekanntmachung des Hinweises auf die Patenterteilung: **11.02.2009**
(21) Anmeldenummer: 00114501.0
(22) Anmeldetag: 06.07.2000
(51) Int. Cl.: C12N 15/77, C12N 15/70, C12N 15/53, C12N 9/02, C12N 15/54, C12N 9/10, C12N 15/63, C12N 1/21, C12P 13/08

(54) **L-Lysin produzierende coryneforme Bakterien und Verfahren zur Herstellung von L-Lysin**
L-lysine producing coryneform bacteria and methods for the production of L-lysine
Bactéries corynéformes produisant L-lysine et procédés pour la production de L-lysine

(30) Priorität: 07.07.1999 DE 19931317
(43) Veröffentlichungstag der Anmeldung: 10.01.2001
(73) Patentinhaber: Evonik Degussa GmbH, 45128 Essen (DE); Forschungszentrum Jülich GmbH, 52425 Jülich (DE)
(72) Erfinder: Möckel, Bettina, Dr., 40597 Düsseldorf (DE); Pfefferle, Walter, Dr., 33790 Halle (Westf.) (DE); Kreutzer, Caroline, 49326 Melle (DE); Hans, Stephan, 49078 Osnabrück (DE); Rieping, Mechthild, Dr., 33619 Bielefeld (DE); Eggeling, Lothar, Dr., 52428 Jülich (DE); Sahm, Hermann, Prof., 52428 Jülich (DE); Patek, Miroslav, Dr., 10600 Praha 10 (CZ)

(56) Entgegenhaltungen:
- EP-A- 0 435 132
- EP-A- 0 854 189
- DE-A- 19 548 222
- DE-A- 19 831 609
- DATABASE EMBL [Online] Accession No. X67737, 1. April 1993 (1993-04-01) "C. glutamicum dapB gene for dihydrodipicolinate reductase" XP002151598

## Beschreibung

Die Erfindung betrifft L-Lysin produzierende Stämme coryneformer Bakterien mit überexprimiertem lysE-Gen (Lysin-Export-Carrier-Gen) in denen zusätzliche das einen mutierten Promoter enthaltende dapA-Gen (Dihydrodipicolinat-Synthase-Gen) und gegebenenfalls das lysC-Gen (Aspartat-Kinase-Gene) oder das dapB-Gen (Dihydrodipicolinat-Reduktase-Gen) insbesondere aber das lysC-Gen (Aspartat-Kinase-Gen) überexprimiert werden, und ein Verfahren zur Herstellung von L-Lysin.

### Stand der Technik

L-Lysin ist eine kommerziell bedeutende L-Aminosäure, die insbesondere als Futtermittelzusatz in der Tierernährung Anwendung findet. Der Bedarf steigt in den letzten Jahren ständig an.

L-Lysin wird fermentativ mit L-Lysin produzierenden Stämmen coryneformer Bakterien insbesondere mit Corynebacterium glutamicum hergestellt. Wegen der großen Bedeutung dieses Produktes wird ständig an der Verbesserung des Herstellverfahrens gearbeitet. Verfahrensbesserungen können fermentationstechnische Maßnahmen wie z.B. Rührung und Versorgung mit Sauerstoff, oder die Zusammensetzung der Nährmedien wie z.B. die Zuckerkonzentration während der Fermentation, oder die Aufarbeitung zur Produktform durch z.B Ionenaustauschchromatographie oder die intrinsischen Leistungseigenschaften des Mikrorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. S-(2-Aminoethyl)-Cystein oder auxotroph für Aminosäuren wie z.B. L-Leucin sind und L-Lysin produzieren.

Seit einigen Jahren werden ebenfalls Methoden der rekombinanten DNA-Technik zur Stammverbesserung L-Lysin produzierender Stämme von Corynebacterium glutamicum eingesetzt, indem man einzelne Biosynthesegene amplifiziert und die Auswirkung auf die L-Lysin-Produktion untersucht.

So wird in EP-A- 0 088 166 über die Leistungssteigerung nach Amplifikation eines Resistenz gegen Aminoethylcystein vermittelnden DNA-Fragmentes berichtet. In EP-B- 0 387 527 wird über die Leistungssteigerung nach Amplifikation eines für eine "feed back" resistente Aspart-Kinase kodierenden lysC-Allels berichtet. In EP-B-0 197 335 wird über die Leistungssteigerung nach Amplifikation des für die Dihydrodipicolinat-Synthase kodierenden dapA-Gens berichtet. In EP-A-0 219 027 wird wird über die Leistungssteigerung nach Amplifikation des für die Aspartatsemialdehyd-Dehydrogenase kodierenden asd-Gens berichtet. Pisabarro et al. (Journal of Bacteriology, 175(9), 2743-2749, (1993)) beschreiben das für die Dihydrodipicolinat-Reduktase kodierende dapB-Gen.

Weiterhin wurde auch die Wirkung der Amplifikation von Genen des Primärstoffwechsels auf die Produktion von L-Lysin untersucht. So wird in EP-A-0 219 027 über die Leistungssteigerung nach Amplifikation des für die Aspartat-Aminotransferase kodierenden aspC-Gens berichtet. In EP-B-0 143 195 und EP-B-0 358 940 wird über die Leistungssteigerung nach Amplifikation des für die Phosphoenolpyruvat-Carboxylase kodierenden ppc-Gens berichtet. In der Offenlegungsschrift DE-A-198 31 609 wird über die Leistungssteigerung nach Amplifikation des für die Pyruvat-Carboxylase kodierenden pyc-Gens berichtet.

Schließlich wird in der Offenlegungsschrift DE-A-195 48 222 beschrieben, daß eine gesteigerte Aktivität des vom lysE-Gen kodierten L-Lysin-Exportcarriers die Lysin-Produktion fördert.

Neben diesen Ansätzen zur Amplifikation eines einzelnen Gens wurden auch Ansätze verfolgt, zwei oder mehrere Gene gleichzeitig zu amplifizieren, und dadurch die L-Lysin-Produktion in coryneformen Bakterien zu verbessern. So wird in der Offenlegungsschrift DE-A-38 23 451 über die Leistungssteigerung nach gleichzeitiger Amplifikation des asd-Gens und des dapA-Gens von Escherichia coli berichtet. Aus der Offenlegungsschrift DE-A-39 43 117 ist die Leistungssteigerung nach gleichzeitiger Amplifikation eines für eine "feed back" resistente kodierenden lysC-Allels und des dapA-Gens mittels des Plasmides pJC50 bekannt. In der EP-A-0 841 395 wird insbesondere über die Leistungssteigerung bei gleichzeitiger Amplifikation eines für eine "feed back" resistente kodierenden lysC-Allels und des dapB-Gens berichtet; durch zusätzliche Amplifikation der Gene dapB, lysA und ddh konnten weitere Verbesserungen erzielt werden. In der EP-A-0 854 189 wird die Leistungssteigerung bei gleichzeitiger Amplifikation eines für eine "feed back" resistente kodierenden lysC-Allels, des dapA-Gens, des dapB-Gens, des lysA-Gens und des aspC-Gens beschrieben. In der EP-A-0 857 784 wird insbesondere über die Leistungssteigerung bei gleichzeitiger Amplifikation eines für ein "feed back" resistentes kodierendes lysC-Allel und des lysA-Gens berichtet; durch zusätzliche Amplifikation des ppc-Gens konnte eine weitere Verbesserung erzielt werden.

Aus der Vielzahl der im Stand der Technik beschriebenen Verfahren wird deutlich, daß ein Bedarf an der Entwicklung neuer Ansätze und der Verbesserung bestehender Verfahren zur Lysin-Produktion mit coryneformen Bakterien besteht.

### Aufgabe der Erfindung

Die Aufgabe der Erfindung besteht darin, mit neuen Maßnahmen verbesserte L-Lysin produzierende Stämme coryneformer Bakterien bereitzustellen.

### Beschreibung der Erfindung

L-Lysin ist eine kommerziell bedeutende L-Aminosäure, die insbesondere als Futtermittelzusatz in der Tierernährung Anwendung findet.

Wenn im folgenden Text L-Lysin oder Lysin erwähnt werden, so sind damit nicht nur die Base sondern auch die entsprechenden Salze wie z.B. Lysin-Hydrochlorid oder Lysin-Sulfat gemeint.

Gegenstand der Erfindung sind L-Lysin produzierende Stämme coryneformer Bakterien mit überexprimiertem lysE-Gen (Lysin-Export-Carrier-Gen), in denen zusätzlich das dapA-Gen (Dihydrodipicolinat-Synthase-Gen) überexprimiert wird, wobei der Promoter des dapA-Gens die Mutationen MC 20 oder MA16 aufweist, wobei gegebenenfalls das lysC-Gen (Aspartat-Kinase-Gen) oder dapB-Gen (Dihydrodipicolinat-Reduktase-Gen) überexprimiert wird.

Weiterhin wurde die neue DNA-Sequenz gefunden, die stromaufwärts (5'-Bereich) des dapB-Gens liegt, welche die -35-Region des dapB-Promotors trägt und vorteilhaft für die Expression des dapB-Gens ist. Sie ist als SEQ-ID-No. 1 dargestellt.

Es wird daher ebenfalls eine entsprechende replizierbare DNA beschrieben mit der Nukleotidsequenz, gezeigt in SEQ-ID-No. 1.

Die Mutationen MC20 oder MA16 des dapA-Promotors sind dargestellt in SEQ-ID-No. 5 und SEQ-ID-No. 6, hinterlegt unter DSM 12868 bzw. DSM 12867.

Eine weiterer Gegenstand der Beschreibung sind L-Lysin produzierende Stämme coryneformer Bakterien mit verstärktem lysE-Gen, die dadurch gekennzeichnet sind, daß zusätzlich gleichzeitig das dapA- und das dapB-Gen verstärkt, insbesondere überexprimiert werden.

Schließlich sind ebenso Gegenstand der Beschreibung L-Lysin produzierende Stämme coryneformer Bakterien mit verstärktem lysE-Gen dadurch gekennzeichnet, daß zusätzlich gleichzeitig das dapA-, das lysC-Gen verstärkt, insbesondere überexprimiert werden.

Der Begriff "Verstärkung" beschreibt in diesem Zusammenhang die Erhöhung der intrazellulären Aktivität eines oder mehrerer Enzyme in einem Mikroorganismus, die durch die entsprechende DNA kodiert werden, indem man die Kopienzahl des(der) Gene erhöht, einen starken Promotor verwendet oder ein Gen verwendet, das für ein entsprechendes Enzym mit einer hohen Aktivität kodiert und gegebenenfalls diese Maßnahmen kombiniert.

Beansprucht wird auch ein Verfahren zur Herstellung von L-Lysin durch Fermentation dieser coryneformen Bakterien.

Die Mikroorganismen, die Gegenstand der vorliegenden Erfindung sind, können L-Lysin aus Glucose, Saccharose, Lactose, Fructose, Maltose, Melasse, Stärke, Cellulose oder aus Glycerin und Ethanol herstellen, insbesondere aus Glucose oder Saccharose. Es handelt sich um coryneforme Bakterien insbesondere der Gattung Corynebacterium. Bei der Gattung Corynebacterium ist insbesondere die Art Corynebacterium glutamicum zu nennen, die in der Fachwelt für ihre Fähigkeit bekannt ist, Aminosäuren zu produzieren. Zu dieser Art gehören Wildtypstämme wie z. B. Corynebacterium glutamicum ATCC13032, Brevibacterium flavum ATCC14067, Corynebacterium melassecola ATCC17965 und davon abgeleitete Stämme bzw. Mutanten. Beispiele für L-Lysin produzierende Mutanten coryneformer Bakterien sind beispielsweise
Corynebacterium glutamicum FERM-P 1709
Brevibacterium flavum FERM-P 1708
Brevibacterium lactofermentum FERM-P 1712
Brevibacterium flavum FERM-P 6463
Brevibacterium flavum FERM-P6464
Corynebacterium glutamicum DSM 5714
Corynebacterium glutamicum DSM 12866

Die förderliche Wirkung der Überexpression des lysE-Gens auf die Produktion von L-Lysin ist aus der Offenlegungsschrift DE-A- 195 48 222 bekannt.

Die zusätzliche verstärkte Expression des dapB-Gens, oder des pyc-Gens, oder insbesondere eine zusätzlich verstärkte Expression eines für eine "feed back" resistente Aspartatkinase kodierenden lysC-Allels, oder insbesondere eine zusätzlich verstärkte Expression des dapA-Gens verbessert die Produktion von L-Lysin.

Die Erfinder fanden weiterhin, dass bei gegebener Überexpression des lysE-Gens die gleichzeitige, zusätzlich verstärkte Expression des dapA- mit dem erfindungsgemäß mutierten Promoter und des dapB-Gens weitere Vorteile für die Produktion von L-Lysin bringt.

Es wird eine entsprechende replizierbare DNA beschrieben mit der Nukleotidsequenz, gezeigt in SEQ-ID-No. 1.

Von Vorteil ist ebenfalls bei gegebener Überexpression des lysE-Gens die gleichzeitige, zusätzlich verstärkte Expression des dapA-, und des lysC-Allels, wobei das dapA-Gen den erfindungsgemäß mutierten Promoter aufweist.

Zur Erzielung einer Verstärkung (Überexpression) erhöht man z. B. die Kopienzahl der entsprechenden Gene oder mutiert die Promotor- und Regulationsregion oder die Ribosomenbindungsstelle, die sich stromaufwärts des Strukturgens befindet. In gleicher Weise wirken Expressionskassetten, die stromaufwärts des Strukturgens eingebaut werden. Durch induzierbare Promotoren ist es zusätzlich möglich die Expression im Verlaufe der fermentativen L-Lysin-Bildung zu steigern. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte liegen dabei entweder in Plasmiden (Pendelvektoren) mit unterschiedlicher Kopienzahl vor oder sind im Chromosom integriert und amplifiziert. Alternativ kann weiterhin eine Überexpression der betreffenden Gene durch Veränderung der Medienzusammen-setzung und Kulturführung erreicht werden.

Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift EPS 0 472 869, im US Patent 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in der Patentanmeldung WO 96/15246, bei Malumbres et al. (Gene 134, 15-24 (1993)), in der japanischen Offenlegungsschrift JP-A-10-229891, bei Jensen und Hammer (Biotechnology and Bioengineering 58, 191-195 (1998)) oder im Handbuch "Manual of Methods for General Bacteriology der American Society for Bacteriology (Washington D.C., USA, 1981 und in bekannten Lehrbüchern der Genetik und Molekularbiologie.

Die erfindungsgemäss verwendeten Gene von Corynebacterium glutamicum sind beschrieben und können mit bekannten Methoden isoliert bzw. hergestellt oder synthetisiert werden.

Methoden zur ortsspezifischen Mutagenese sind unter anderem bei Higuchi et al. (Nucleic Acids Research 16: 7351 - 7367 (1988)) oder bei Silver et al. im Handbuch von Innis, Glefand und Sninsky (eds.): PCR Strategies (Academic Press, London, UK, 1995) beschrieben.

Zur Isolierung eines interessierenden Gens von C. glutamicum wird zunächst eine Genbank dieses Mikrorganismus in z.B. E. coli oder gegebenenfalls auch in C. glutamicum angelegt. Das Anlegen von Genbanken ist in allgemein bekannten Lehrbüchern und Handbüchern niedergeschrieben. Als Beispiel seien das Lehrbuch von Winnacker: Gene und Klone, Eine Einführung in die Gentechnologie (Verlag Chemie, Weinheim, Deutschland, 1990) oder das Handbuch von Sambrook et al.: Molecular Cloning, A Laboratory Manual (Cold Spring Harbor Laboratory Press, 1989) genannt. Bathe et al. (Molecular and General Genetics, 252: 255-265, 1996) beschreiben eine Genbank von C. glutamicum ATCC13032, die mit Hilfe des Cosmidvektors SuperCos I (Wahl et al., 1987, Proceedings of the National Academy of Sciences USA, 84:2160-2164) im E.coli K-12 NM554 ( Raleigh et al., 1988, Nucleic Acids Research 16:1563-1575) angelegt wurde. Börmann et al. (Molecular Microbiology 6(3), 317-326)) wiederum beschreiben eine Genbank von C. glutamicum ATCC13032 unter Verwendung des Cosmids pHC79 (Hohn und Collins, Gene 11, 291-298 (1980)). Zur Herstellung einer Genbank von C. glutamicum in E. coli können auch Plasmide wie pBR322 (Bolivar, Life Sciences, 25, 807-818 (1979)) oder pUC19 (Norrander et al., 1983, Gene, 26: 101-106) verwendet werden. In gleicher Weise können auch Pendelvektoren wie z.B. pJC1 (Cremer et al., Molecular and General Genetics 220, 478-480 (1990)) oder pEC5 (Eikmanns et al., 1991, Gene 102, 93-98), die in E. coli und C. glutamicum replizieren, verwendet werden. Als Wirte eignen sich besonders solche Stämme, die restriktions- und/oder rekombinationsdefekt sind. Ein Beispiel hierfür ist der E. coli Stamm DH5αmcr, der von Grant et al. (Proceedings of the National Academy of Sciences USA, 87 (1990) 4645-4649) beschrieben wurde. Ein anderes Beispiel hierfür sind die restriktionsdefekten C. glutamicum Stämme RM3 und RM4, die bei Schäfer et al. (Applied and Environmental Microbiology 60(2), 756-759, (1994)) beschrieben sind.

Die Genbank wird anschließend in einen Indikatorstamm durch Transformation (Hanahan, Journal of Molecular Biology 166, 557-580, 1983) oder Elektroporation (Tauch et.al., 1994, FEMS Microbiological Letters, 123:343-347) überführt. Der Indikatorstamm zeichnet sich dadurch aus, dass er eine Mutation in dem interessierenden Gen besitzt, die einen detektierbaren Phänotyp z.B. eine Auxotrophie hervorruft. Die Indikatorstämme bzw. Mutanten sind aus publizierten Quellen oder Stammsammlungen wie z.B. dem Genetic Stock Center der Yale University (New Haven, Connecticut, USA) erhältlich oder werdem gegebenfalls selbst hergestellt. Als Beispiel eines derartigen Indikatorstammes sei der meso-Diaminopimelinsäure bedürftige E. coli Stamm RDA8 (Richaud et al. C. R. Acad. Sci. Paris Ser. III 293: 507-512 (1981)) genannt, der eine Mutation (dapA::Mu) im dapA-Gen trägt.

Nach Transformation des Indikatorstammes mit einem rekombinanten Plasmid, welches das interessierende Gen trägt, und Expression des betreffenden Gens, wird der Indikatorstamm bezüglich der entsprechenden Eigenschaft prototroph. Vermittelt das klonierte DNA-Fragment Resistenz z.B. gegen einen Antimetaboliten wie S-(2-Aminoethyl)-Cystein kann die Identifizierung des das rekombinante Plasmid tragenden Indikatorstammes durch Selektion auf entsprechend supplementierten Nährböden erfolgen

Bei bekannter bzw. in einer Datenbank zugänglichen Nukleotidsequenz der interessierenden Genregion kann die chromosomale DNA mit bekannten Methoden wie z.B. bei Eikmanns et al. (Microbiology 140, 1817-1828, (1994)) beschrieben, isoliert und das betreffende Gen durch die Polymerase-Kettenreaktion (PCR) unter Verwendung geeigneter Primer synthetisiert und in einen geeigneten Plasmidvektor wie z.B. pCRIITOPO der Firma Invitrogen (Groningen, Niederlande) kloniert werden. Eine Zusammenfassung zur PCR-Methodik kann dem Buch von Newton und Graham: PCR (Spektrum Akademischer Verlag, Heidelberg, Deutschland, 1994) entnommen werden.

Öffentlich zugängliche Datenbanken für Nukleotidsequenzen sind beispielsweise die der European Molecular Biologies Laboratories (EMBL, Heidelberg, Deutschland) oder die des National Center for Biotechnology Information (NCBI, Behesda, MD, USA).

Die Isolierung und Klonierung des lysE-Gens von C. glutamicum ATCC13032 ist ebenso wie die Nukleotidsequenz in der Offenlegungsschrift DE-A-195 48 222 beschrieben.

Die Isolierung, Klonierung und Sequenzierung des dapA-Gens verschiedener Stämme von C. glutamicum sind bei Cremer et al. (Molecular and General Genetics 220:478-480 (1990)), bei Pisabarro et al.(Journal of Bacteriology 175:2743-2749 (1993)) und bei Bonnassie et al. (Nucleic Acids Research 18:6421 (1990) beschrieben. Die Nukleotidsequenz des dapA-Gens ist unter der accession number X53993 zugänglich.

Die Isolierung, Klonierung und Sequenzierung des dapB-Gens von Brevibacterium lactofermentum ist bei Pisabarro et al.(Journal of Bacteriology 175:2743-2749 (1993)) beschrieben. Die Nukleotidsequenz des dapB-Gens ist unter der accession number x67737 zugänglich.

Die Isolierung, Klonierung und Sequenzierung des lysC-Gens und von lysC-Allelen, die für eine "feed back"-resistente Aspartatkinase kodieren wird von mehreren Authoren berichtet. So berichten Kalinowski et al. (Molecular and General Genetics 224:317-324 (1990)) über das lysC-Allel des Stammes C. glutamicum DM58-1. In der DE-A-39 43 117 wird über die Klonierung des lysC-Allels des C. glutamicum Stammes MH20 berichtet. Follettie et al. (Journal of Bacteriology 175:4096-4103 (1993)) berichten über das lysC-Allel des Stammes C. flavum N13, das dort als ask bezeichnet wird. Kalinowski et al. (Molecular Microbiology 5,1197-1204 (1991) berichten über das lysC-Gen von C. glutamicum ATCC13032. Die Nukleotidsequenzen des lysC-Gens und verschiedener lysC-Allele sind unter anderem unter der accession number X57226, E06826 zugänglich.

Die auf diese Weise gewonnenen Gene können dann unter anderem in Plasmidvektoren wie z.B. pJC1 (Cremer et al., Molecular and General Genetics 220, 478-480 (1990)) oder pEC5 (Eikmanns et al.,1991, Gene 102, 93-98) einzeln oder in geeigneten Kombinationen eingebaut und in gewünschten Stämmen coryneformer Bakterien z.B. den Stamm MH20-22B (Schrumpf et al., Applied Microbiology and Biotechnology 37:566-571 (1992)) durch Transformation wie z.B. bei Thierbach et al. (Applied Microbiology and Biotechnology 29, 356-362 (1988) oder Elektroporation wie z.B. bei Dunican und Shivnan (Bio/Technology 7,1067-1070 (1989)) beschrieben, eingebaut und zur Expression gebracht werden. Es ist gleichfalls möglich den auszuwählenden Stamm mit zwei Plasmidvektoren, die jeweils das betreffende Gen oder die betreffenden Gene enthalten, zu transformieren und dadurch die vorteilhafte gleichzeitig verstärkte Expression zweier oder mehrerer Gene zusätzlich zur bekannten Verstärkung des lysE-Gens zu erzielen.

Beispiele für derartige Stämme sind (nicht Teil der Erfindung):
- der Stamm MH20-22B/pJC33/pEC7lysE bei dem das lysE- und das lysC-Gen gleichzeitig verstärkt exprimiert sind, oder
- der Stamm MH20-22B/pJC50/pEC7lysE bei dem das lysE-, das lysC- und das dapA-Gen gleichzeitig verstärkt exprimiert sind, oder
- der Stamm MH20-22B/pJC23/pEC7lysE bei dem das lysE- und das dapA-Gen gleichzeitig verstärkt exprimiert sind, oder
- der Stamm MH20-22B/pJC23/pEC7dapBlysE bei dem das lysE-, das dapA-Gen und das dapB gleichzeitig verstärkt exprimiert sind

Die erfindungsgemäß hergestellten Mikroorganismen können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der L-Lysin-Produktion kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Mikroorganismen genügen. Beschreibungen von Kulturmedien verschiedenener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z.B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoff haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden setzt man dem Medium gegebenenfalls geeignete selektiv wirkende Stoffe wie z.B. Antibiotika hinzu. Um aerobe Bedingungen aufrechtzuerhalten, werden Sauerstoff oder Sauerstoff haltige Gasmischungen wie z.B. Luft in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum an L-Lysin gebildet hat. Dieses Ziel wird normalerweise innerhalb von 10 Stunden bis 160 Stunden erreicht.

Die Konzentration an gebildetem L-Lysin kann mit Hilfe von Aminosäure-Analysatoren durch Jonenaustauschchromatographie und Nachsäulenreaktion mit Ninhydrindetektion, wie bei Spackmann et al. (Analytical Chemistry 30, 1190 (1958)) beschrieben, bestimmt werden.

Folgende Mikroorganismen wurden bei der Deutschen Sammlung für Mikrorganismen und Zellkulturen (DSMZ, Braunschweig, Deutschland) gemäß Budapester Vertrag hinterlegt: (Teil der Beschreibung)
- Escherichia coli K-12 Stamm DH5α/pEC7lysE als DSM 12871
- Escherichia coli K-12 Stamm DH5α/pEC7dapBlysE als DSM 12875
- Corynebacterium glutamicum Stamm DSM5715/pJC23 als DSM 12869
- Corynebacterium glutamicum Stamm DSM5715aecD::dapA(MA16) als DSM12867
- Corynebacterium glutamicum Stamm DSM5715aecD::dapA(MC20) als DSM12868

### Beispiele

### Beispiel 1

### Gewinnung der für lysE codierenden DNA

Aus dem Stamm ATCC 13032 wurde mit den üblichen Methoden (Eikmanns et al., Microbiology 140: 1817 - 1828 (1994)) chromosomale DNA isoliert. Mit Hilfe der Polymerase-Kettenreaktion (PCR) wurde ein DNA Fragment amplifiziert, das das lysE Gen trägt. Aufgrund der für C. glutamicum bekannten Sequenz des lysE Gens (Vrljic et al., Molecular Microbiology 22(5), 815 - 826 (1996)) (Accession number X96471) wurden die folgenden Primer-Oligonukleotide für die PCR ausgewählt:
LysBam1:
   5' CTC GAG AGC (GGA TCC) GCG CTG ACT CAC C 3'
LysBam2:
   5' GGA GAG TAC GGC (GGA TCC) ACC GTG ACC 3'

Die dargestellten Primer wurden von der Firma MWG Biotech (Ebersberg, Deutschland) synthetisiert und nach der Standard-PCR-Methode von Innis et al. (PCR protocols. A guide to methods and applications, 1990, Academic Press) die PCR Reaktion durchgeführt. Die Primer ermöglichen die Amplifizierung eines ca. 1,1 kb großen DNA-Fragmentes, welches das lysE Gen trägt. Außerdem enthalten die Primer die Sequenz für die Schnittstelle der Restriktionsendonuklease BamHI , die in der oben dargestellten Nukleotidabfolge durch Klammern markiert ist.

Das amplifizierte DNA Fragment von ca. 1,1 kb, welches das lysE Gen trägt, wurde mittels Elektrophorese in einem 0,8%igen Agarosegel identifiziert, aus dem Gel isoliert und mit dem QIAquick Gel Extraction Kit (Cat. No. 28704) der Firma Quiagen (Hilden, Deutschland) aufgereinigt. Anschließend erfolgte die Ligation des Fragmentes mittels T4 DNA Ligase der Firma Boehringer Mannheim (Mannheim, Deutschland) in den Vektor pUC18 (Norrander et al., Gene (26) 101 - 106 (1983)). Hierzu wurde der Vektor pUC18 mit der Restriktionsendonuklease SmaI vollständig geschnitten und mit alkalischer Phosphatase (Alkaline Phosphatase, Boehringer Mannheim, Mannheim, Deutschland) behandelt. Der Ligationsansatz wurde in den E. coli Stamm DH5α (Hanahan, In: DNA cloning. A practical approach. Vol.I. IRL-Press, Oxford, Washington DC, USA) transformiert. Die Selektion von Plasmid-tragenden Zellen erfolgte durch Ausplattieren des Transformationsansatzes auf LB Agar (Sambrook et al., Molecular cloning: a laboratory manual. 2nd Ed. Cold Spring Harbor Laboratory Press, Cold Spring Harbor, N.Y.), der mit 50 mg/l Ampicillin supplementiert worden war. Plasmid DNA wurde aus einer Transformante isoliert, durch Behandlung mit dem Restriktionsenzym BamHI mit anschließender Agarosegel-Elektrophorese überprüft. Das Plasmid wurde pUC18lysE genannt.

### Beispiel 2

### Gewinnung von dapB

Chromosomale DNA wurde wie in Beispiel 1 angegeben aus dem Corynebacterium glutamicum Stamm ATCC 13032 isoliert. Die Sequenz des dapB Gens als solcher aus Corynebacterium glutamicum ist bekannt (Accession number X67737). Jedoch umfaßt die veröffentlichte DNA Sequenz nur 56 bp vor dem Translationsstart. Daher wurde zusätzlich der 5'Bereich vor dem Translationsstart sequenziert.

Dazu wurde mit dem Plasmid pJC25 (EP-B 0 435 132) und unter Verwendung eines Primer-Oligonukleotides, welches in der Region der bekannten dapB Sequenz (Accession number X67737) bindet, die Sequenzierung durchgeführt. Die Sequenz des verwendeten Sequenzierungsprimers war:
5' GAA CGC CAA CCT TGA TTC C 3'

Die Sequenzierung erfolgte nach der bei Sanger et al., Proc. Natl. Acad. Sci. USA, (74) 5463 - 5467 (1977) beschriebenen Kettenabbruch-Methode. Die Sequenzierungsreaktion wurde mit Hilfe des AutoRead Sequencing Kit (Pharmacia, Freiburg) durchgeführt. Die elektrophoretische Analyse und Detektion der Sequenzierprodukte wurde mit dem A.L.F.-DNA-Sequenzierer der Firma Pharmacia (Freiburg, Deutschland) durchgeführt.

Die erhaltene DNA Sequenz wurde zur Auswahl eines zweiten Primers verwendet, um weitere Sequenzdaten vor dem Transkriptionstart zu erhalten. Dazu wurde folgender Primer ausgewählt:
5' CTT TGC CGC CGT TGG GTT C 5'

Die Sequenzierungsreaktion erfolgte wie oben beschrieben. Die neue Sequenz stromaufwärts des dapB-Gens ist als SEQ ID NO 1 dargestellt. Die Sequenz inklusive der Nukleotidabfolge des dapB-Gens ist als SEQ ID NO 2 dargestellt.

Das dapB Gen wurde mit Hilfe der Polymerase-Kettenreaktion amplifiziert. Dazu wurden von der Firma MWG Biotech zwei Primer-Oligonukleotide synthetisiert die aufgrund der bekannten DNA-Sequenz des dapB Gens ausgewählt wurden:
P-dap:
   5' (AAG CTT) AGG TTG TAG GCG TTG AGC 3'
dapall:
   5' TTA ACT TGT TCG GCC ACA GC 3'

Der 5' Primer (Primer P-dap) enthält eine HindIII Schnittstelle, die in der oben dargestellten Sequenz durch Klammern markiert ist. Die Durchführung der PCR erfolgte gemäß Beispiel 1. Auf diese Weise wurde ein ca 1,1 kb DNA-Fragment amplifiziert, welches das dapB Gen trägt und an einem Ende eine Schnittstelle für die Restriktionsendonuklease HindIII enthält. Das erhaltene PCR Fragment wurde aus dem 0,8%igen Agarosegel aufgereinigt (QIAquick Gel Extraction Kit der Firma Qiagen, Hilden, Deutschland)) und mit dem TOPO TA Cloning Kit (Invitrogen, Leek, Niederlande, Cat. No K4550-01) in den Klonierungsvektor pCR2.1TOPO (Invitrogen, Leek, Niederlande) kloniert. Der Ligationsansatz wurde in den E. coli Stamm TOP10F' der Firma Invitrogen, transformiert, der Transformationsansatz auf Kanamycin (50 mg/l) haltigem LB-Agar mit IPTG (0,16mM) und X-Gal (64 mg/l) ausplattiert und Kanamycin-resistente und weiß gefärbte Kolonien isoliert. Plasmid-DNA wurde aus einer Transformante mit Hilfe des QIAprep Spin Miniprep Kit der Firma Qiagen isoliert und durch Restriktionsspaltung mit dem Enzym HindIII und anschließender Agarosegel-Elektrophorese überprüft. Die DNA Sequenz des amplifizierten DNA Fragmentes wurde durch Sequenzierung überprüft. Die Sequenz des PCR Produktes stimmt mit der in SEQ ID NO 1 dargestellten Sequenz überein. Das erhaltene Plasmid wurde pCR2.1TOPOdapB genannt.

### Beispiel 3

### Klonierung von lysE im Vektor pEC7

Das lysE tragende Fragment aus dem Plasmid pUC18lysE (Beispiel 1) wurde wie im folgenden beschrieben, in den Vektor pEC7 inseriert. Der Vektor pEC7 basiert auf dem E. coli - C. glutamicum Shuttle Vektor pEC5 (Eikmanns et al., 1991, 102: 93 - 98). Aus dem Plasmid pEC5 wurde diejenige BamHI Schnittstelle, die nicht im Polylinker positioniert ist, auf folgende Weise entfernt: Das Plasmid pEC5 wurde partiell mit dem Restriktionsenzym BamHI gespalten. Das ca 7,2 kb große DNA Fragment wurde aus dem Agarosegel isoliert und die überstehenden Enden mit der Klenow-Polymerase (Boehringer Mannheim) aufgefüllt. Das so erhaltene DNA Fragment wurde ligiert (T4-Ligase, Boehringer Mannheim). Der Ligationsansatz wurde in den E. coli Stamm DH5α transformiert und Kanamycin resistente Kolonien auf LB Agar mit Kanamycin (50 mg/l) isoliert. Plasmid-DNA wurde aus einer Transformante isoliert (QIAprep Spin Miniprep Kit der Firma Qiagen) und durch Restriktionsspaltung mit den Restriktionsenzymen BamHI und PstI überprüft. Das so erhaltene Plasmid wurde pEC6 genannt.

Das Plasmid pEC6 wurde mit dem Restriktionsenzym XhoI vollständig gespalten. Ein DNA-Fragment, welches den trp-Terminator trägt, wurde mit dem Vektor DNA Fragment ligiert (T4-Ligase, Boehringer Mannheim). Der Ligationsansatz wurde in den E. coli Stamm DH5α transformiert und Chloramphenicol resistente Kolonien auf LB Agar mit Chloramphenicol (50 mg/l) isoliert. Plasmid-DNA wurde aus einer Transformante isoliert (QIAprep Spin Miniprep Kit der Firma Qiagen) und durch Restriktionsspaltung mit den Restriktionsenzymen BamHI und XhoI überprüft. Das so erhaltene Plasmid wurde pEC7 genannt.

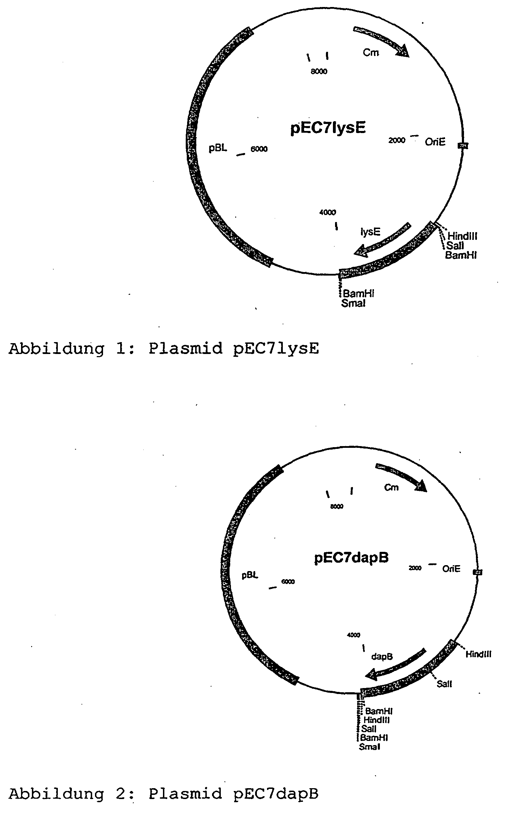

Das in Beipiel 1 beschriebene Plasmid pUC18lysE wurde mit dem Restriktionsenzym BamHI vollständig verdaut und das 1,1 kb große BamHI Fragement mit dem lysE Gen wie in Beispiel 1 isoliert. Ebenso wurde der Vektor pEC7 mit dem Restriktionsenzym BamHI vollständig geschnitten und mit alkalischer Phosphatase behandelt. Das BamHI Vektorfragment und das BamHI lysE Fragment wurden ligiert (Rapid DNA Ligation Kit, Boehringer Mannheim), und in den E. coli Stamm DH5α transformiert. Plasmid-tragende Transformanten wurden auf Chloramphenicol-haltigem LB Agar (10 mg/l) selektioniert. Plasmid-DNA wurde isoliert (QIAprep Spin Miniprep Kit, Qiagen) und durch Restriktionsspaltung mit dem Enzym BamHI überprüft. Das so erhaltene Plasmid wurde pEC7lysE (Abbildung 1)genannt. Der durch Transformation des Plasmides pEC7lysE in den E. coli Stamm DH5α erhaltene Stamm wurde DHSα/pEC7lysE genannt.

### Beispiel 4

### Klonierung von dapB im Vektor pEC7

Aus dem Plasmid pCR2.1TOPOdapB (aus Beispiel 2) wurde ein ca. 1,1 kb großes DNA Fragment, welches das dapB Gen trägt isoliert. Das Plasmid pCR2.1TOPOdapB wurde dazu mit dem Restriktionsenzym HindIII vollständig verdaut und das ca 1,1 kb große DNA Fragment mit dem dapB Gen isoliert.

Das so erhaltene dapB-tragende DNA Fragment wurde mit dem Vektor pEC7 (Beispiel 3) ligiert (T4 DNA Ligase, Boehringer Mannheim), der ebenfalls mit dem Restriktionsenzym HindIII vollständig verdaut und mit alkalischer Posphatase (Alkaline Phosphatase, Boehringer Mannheim) behandelt worden war. Der Ligationsansatz wurde in den E. coli Stamm DH5α transformiert und Kanamycin resistente Kolonien auf LB Agar mit Kanamycin (50 mg/l) isoliert. Plasmid-DNA wurde aus einer Transformante isoliert (QIAprep Spin Miniprep Kit der Firma Qiagen) und durch Restriktionsspaltung mit dem Restriktionsenzym HindIII überprüft. Das so erhaltene Plasmid wurde pEC7dapB (Abbildung 2) genannt. Der erhaltene Escherichia coli Stamm wurde DH5α/pEC7dapB genannt.

### Beispiel 5

### Herstellung eines gleichzeitig dapB und lysE enthaltenden Plasmides

Aus dem Plasmid pCR2.1TOPOdapB, welches das dapB Gen aus C. glutamicum ATCC 13032 enthält, wurde das dapB Gen als HindIII Fragment isoliert. Dazu wurde das Plasmid vollständig mit dem Restriktionsenzym HindIII verdaut und das dapB tragende DNA Fragment aus einem 0,8% igem Agarosegel isoliert (QIAquick Gel Extraction Kit, Qiagen). Außerdem wurde der Vektor pEC7 (Beispiel 3) mit dem Restriktionsenzym HindIII vollständig verdaut und mit alkalischer Phosphatase behandelt. Mit dem so erhaltenen linearen Vektorfragment wurde das 1,1 kb dapB enthaltende Fragment ligiert (T4 Ligase, Boehringer Mannheim) und der Ligationsansatz in den E. coli Stamm DH5α transformiert. Plasmid-tragende Transformanten wurden auf Chloramphenicol-haltigem LB Agar ( 10 mg/l) selektioniert. Plasmid-DNA wurde isoliert (QIAprep Spin Miniprep Kit, Qiagen, Hilden, Deutschland) und durch Restriktionsspaltung mit dem Restriktionsenzym Hind III überprüft.

Das so erhaltene Plasmid wurde pEC7lysEdapB genannt. Dieses Plasmid ist in Escherichia coli und in Corynebacterium autonom replizierbar und verleiht seinem Wirt Resistenz gegenüber dem Antibiotikum Chloramphenicol.

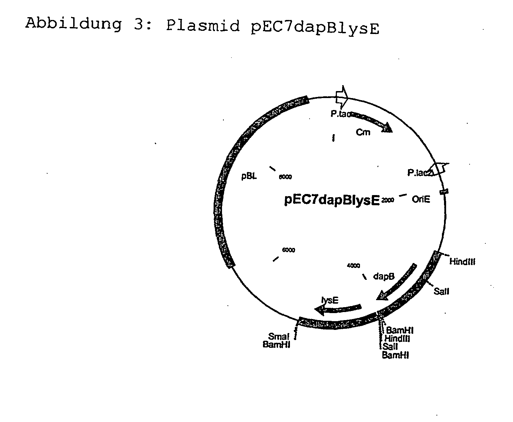

Das Plasmid pEC7lysEdapB enthält, wie in Abbildung 3 dargestellt gleichzeitig das dapB Gen, welches für die Dihyrodipicolinat Reductase kodiert, und das lysE Gen, welches für den Lysinexporter kodiert. Der durch Transformation von E. coli Stamm DH5α mit pEC7lysEdapB erhaltene Stamm wurde DH5α/pEC7lysEdapB genannt.

### Beispiel 6

### Transformation des Stammes MH20-22B mit den Plasmiden pJC1, pJC33 und pJC50

Das Plasmid pJC1 ist ein in Escherichia coli und in Corynebacterium glutamicum replizierbares Plasmid (Cremer et al., 1990, Molecular and General Genetics 220:478 - 480). Davon abgeleitet ist das Plasmid pJC33 (Cremer et al., 1991. Applied and Environmental Microbiology 57 (6) 1746 - 1752) welches das lysC(Fbr) Gen aus dem C. glutamicum Stamm MH20-22B trägt.

Das Plasmid pJC50 basiert ebenfalls auf dem Vektor pJC1. und trägt das lysC FBR Gen aus C. glutamicum MH20-22B und das dapA Gen aus C. glutamicum ATCC 13032 (DE-A-39 43 117).

In den Stamm MH20-22B wurden mit der Elektroporationsmethode (Haynes und Britz, FEMS Microbiology Letters. (61) 329 - 334 (1989))die Plasmide pJC1,pJC33 und pJC50 eingeschleust. Der C. glutamicum Stamm MH20-22B ist ein AEC resistenter Lysinproduzent der unter der Hinterlegungsnummer DSM5715 hinterlegt ist.

Die mit Hilfe der Elektroporation erhaltenen Transformanten wurden auf Selektionsagar (LBHIS Agar (18,5 g/l Brain-Heart Infusion Boullion, 0,5M Sorbitol, 5 g/l Bacto-Trypton, 2,5 g/l Bacto-Yeast-Extract, 5 g/l NaCl, 18 g/l Bacto-Agar)) mit 15 mg/l Kanamycin isoliert. Plasmid DNA wurde nach den üblichen Methoden isoliert (Peters-Wendisch et al., 1998, Microbiology, 144, 915 - 927), mit geeigneten Restritionsendonukleasen geschnitten und überprüft. Die erhaltenen Stämme wurden MH20-22B/pJC1, MH20-22B/pJC33 und MH20-22B/pJC50 genannt.

### Beispiel 7

### Transformation mit den Plasmiden pEC7lysE und pEC7dapBlysE

Im weiteren wurden die im Beipiel 6 hergestellten Stämme verwendet, um sie mit einem zweiten Plasmid zu versehen.

In die beschriebenen Stämme MH20-22B/pJC1, MH20-22B/pJC33 und MH20-22B/pJC50, wurden die Plasmide pEC7lysE und pEC7dapBlys mit der Elektroporationsmethode eingeschleust.

Die transformierten Bakterien werden aufgrund der Antibiotika-Resistenz der enthaltenen Plasmide selektioniert. Die mit Hilfe der Elektroporation erhaltenen Transformanten wurden auf Selektionsagar (LBHIS Agar mit 15 mg/l Kanamycin und 7,5 mg/l Chloramphenicol isoliert. Plasmid DNA wurde isoliert ,mit geeigneten Restriktionsendonukleasen geschnitten und überprüft.

### Beispiel 8 (nicht Teil der Erfindung)

### Herstellung von Lysin

Die in Beispiel 7 erhaltenen verschiedenen C. glutamicum Stämme wurden in einem zur Produktion von Lysin geeigneten Nährmedium kultiviert und der Lysingehalt im Kulturüberstand bestimmt.

Dazu wurden die verschiedenen Stämme zunächst auf Agarplatten mit den entsprechenden Antibiotika (Hirn-Herz Agar mit Kanamycin (25 mg/l), Chloramphenicol (10 mg/l)) 24h bei 33°C inkubiert. Ausgehend von diesen Agarplattenkulturen wurde eine Vorkultur angeimpft (10 ml Medium im 100 ml Erlenmeyerkolben). Als Medium für die Vorkultur wurde das Vollmedium CgIII verwendet. Es wurde Kanamycin (25 mg/l)und Chloramphenicol (10 mg/l) zugesetzt. Die Vorkultur wurde 24 Stunden bei 33°C bei 240 rpm auf dem Schüttler inkubiert. Von dieser Vorkultur wurde eine Hauptkultur angeimpft, so daß die Anfangs OD (660nm) der Hauptkultur 0,2 OD beträgt. Für die Hauptkultur wurde das Medium MM verwendet.

| Medium MM | | |
|---|---|---|
| CSL (Corn Steep Liquor) | 5 g/l | |
| MOPS | 20 g/l | |
| Glucose | 50g/l | (getrennt autoklavieren) |

| Salze: | | |
|---|---|---|
| (NH4)2SO4 | 25 g/l | |
| KH2PO4 . | 0,1 g/l | |
| MgSO4* 7 H2O | 1,0 g/l | |
| CaCl2*2H2O | 10 mg/l | |
| FeSO4 * 7H2O 10 | mg/l | |
| MnSO4*H2O | 5,0mg/l | |
| Biotin | 0,3 mg/l | (sterilfiltriert) |
| Thiamin*HCl | 0,2 mg/l | (sterilfiltriert) |
| CaCO3 | 25 g/l | |

| | | |
|---|---|---|
| CSL, MOPS und die Salzlösung werden mit Ammoniakwasser auf pH 7 eingestellt und autoklaviert. Anschließend werden die sterilen Substrat und Vitaminlösungen zugesetzt, sowie das trocken autoklavierte CaCO3 zugesetzt. | | |

Die Kultivierung erfolgt in 10 ml Volumen in einem 100 ml Erlenmeyerkolben mit Schikanen. Es wurde Kanamycin (25 mg/l)und Chloramphenicol (10 mg/l) zugesetzt. Die Kultivierung erfolgte bei 33°C und 80% Luftfeuchte.

Nach 48 Stunden wurde die OD bei einer Messwellenlänge von 660nm mit dem Biomek 1000 (Beckmann Instruments GmbH, München) ermittelt. Die gebildete Lysinmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt. Der Glukosegehalt wurde mit dem Zuckeranalysator der Firma Skalar Analytik GmbH (Erkelenz, Deutschland) bestimmt.

In Tabelle 1 ist das Ergebnis des Versuchs dargestellt.

**Tabelle-1**

| Stamm | Gen | OD(660) | Lysin-HCl g/l |
|---|---|---|---|
| DSM5715/pJC1/ pEC71ysE | lysE | 9,1 | 11,1 |
| DSM5715/pJC33/ pEC71ysE | lysE, lysC | 8,7 | 12,2 |
| DSM5715/pJC50/ pEC7lysE | lysE, lysC, dapA | 9,1 | 12,7 |
| DSM5715/pJC23/ pEC71ysE | lysE; dapA | 10,2 | 13,3 |
| DSM5715/pJC23/ pEC7dapBlysE | lysE, dapA, dapB | 10,9 | 15,4 |

### Beispiel 9

### Klonierung des aecD Gens im Vektor pUC18

Das Plasmid pSIR21 (Rossol, Dissertation, Universität Bielefeld 1992) wurde mit den Enzymen BglII und EcoRV vollständig gespalten und das 1.4kb große DNA Fragment, welches das aecD Gen (Accessionnumber M89931) (Rossol and Pühler, Journal of Bacteriology, 174 (9), 2968-2977 (1992)) aus C. glutamicum ATCC 13032 enthält, isoliert. Das isolierte DNA Fragment wurde mit dem Plasmid pUC18, das mit den Enzymen BamHI und SmaI vollständig verdaut worden war, mit Hilfe der T4 DNA Ligase wie bei Sambrook et al. (Molecular Cloning: a Laboratory Manual (1989) Cold Spring Harbor Laboratory Press) beschrieben ligiert. Der Ligationsansatz wurde in den E. coli Stamm DH5α transformiert. Die Selektion der Transformanten erfolgte auf Hirn-Herz-Platten mit Ampicillin 100mg/l. Von einer Kolonie wurde Plasmid-DNA isoliert. Das erhaltene Plasmid wurde pUC18::aecD genannt.

### Beispiel 10

### Klonierung des dapA Gens im Plasmid pSP72

Ein dapA Genfragment wird aus dem Plasmid pJC20 (Cremer, J. Dissertation 1989, Universität Düsseldorf) als SphI-BamHI Fragment isoliert. Der Vektor pSP72 (Promega Corporation, USA) wurde mit den Enzymen SphI und BamHI vollständig gespalten und mit alkalischer Phosphatase behandelt. In diesen Vektor wurde das dapA tragende Fragment mit Hilfe der T4-DNA-Ligase ligiert. Die DNA wurde in den E. coli Stamm XL1 Blue (Bullock, Fernandez and Short, BioTechniques (5) 376-379 (1987)) transformiert. Die Selektion der Transformanten erfolgte auf LB Medium mit Ampicillin 100mg/l. Aus einer Transformante wurde Plasmid-DNA isoliert und als pSP72::dapA bezeichnet.

### Beispiel 11

### Mutagenese des dapA Promotors und Herstellung der Plasmide pSP72::dapA(MC20) und pSP72::dapA(MA16)

Für die Mutagenese des Promotorbereiches wurde der Quickchange site directed mutagenesis kit der Firma Stratagene verwendet. Es wurden folgende Primer anhand der vorliegenden dapA Sequenz erstellt und für die Mutagenese eingesetzt:
Für die Herstellung von pSP72::dapA(MC20)
   Primer dap1 für MC20
      CCA AAT GAG AGA TGG TAA CCT TGA ACT CTA TGA GCA
   Primer dap2 für MC20
      GTG CTC ATA GAG TTC AAG GTT ACC ATC TTC CCT CAT TTG G
Für die Herstellung von pSP72::dapA(MA16)
   Primer dap3 für MA16
      CCA AAT GAG GGA AGA AGG TAT AAT TGA ACT CTA TGA GCA
   Primer dap4 für MA16
      GTG CTC ATA GAG TTC AAT TAT ACC TTC TTC CCT CAT TTG G

Die PCR Reaktion erfolgte, wie vom Hersteller (Stratagene) des Quickchange site directed mutagenesis kits angegeben und unter Verwendung des Plasmides pSP72::dapA (aus Beispiel 10) als Template.

Es erfolgte die Transformation der Mutageneseansätze in den E. coli Stamm XL1-Blue. Die Selektion der Transformanten erfolgte auf LB Medium mit Carbenicillin 100mg/l. Aus einer Transformante wurde Plasmid-DNA isoliert, durch BstEII Verdau wurde der Wegfall der BstEII Schnittstelle kontrolliert. Plasmide, die keine BstEII Schnittstelle mehr tragen, wiesen die gewünschte Mutation auf.

Die erhaltenen Plasmide wurden in die dapA defekte E. coli Mutante RDA8 transformiert. Die Transformationsansätze wurden auf LB mit Carbenicillin 100mg/l ausplattiert, um die Komplementation der dapA Mutation zu testen. Aus je einer der Transformanten wurde DNA isoliert und die erhaltenen Plasmide wurden pSP72::dapA(MC20) und pSP72::dapA(MA16) genannt. Es erfolgte die Sequenzierung der Plasmide unter Verwendung der reverse und universal sequencing Primer nach der bei Sanger et al., Proceedings of National Academy of Sciences of the USA, (74) 5463 - 5467 (1977) beschriebenen Kettenabbruch-Methode. Die Sequenzierungsreaktion wurde mit Hilfe des AutoRead Sequencing Kit (Pharmacia, Freiburg) durchgeführt. Die elektrophoretische Analyse und Detektion der Sequenzierprodukte wurde mit dem A.L.F.-DNA-Sequenzierer (Pharmacia, Freiburg) durchgeführt.

### Beispiel 12

### Herstellung der Plasmide pK19mobsacBaecD::dapA(MC20) und pK19mobsacBaecD::dapA(MA16) (Umklonierung der mutagenisierten Fragmente)

Die Plasmide pSP72::dapA(MC20) und pSP72::dapA(MA16) (aus Beispiel 11) wurden mit den Restriktionsenzymen PvuII und SmaI vollständig geschnitten. Die 1450 bp großen PvuII-SmaI Fragmente, welche das dapA Gen mit dem mutierten Promotor MC20 bzw. MA16 tragen, wurden mit der T4-DNA-Ligase in den StuI geschnittenen Vektor pUC18::aecD (aus Beispiel 9) ligiert. Der Ligationsansatz wurde in den E. coli Stamm DH5α transformiert. Die Selektion der Transformanten erfolgte auf LB Medium mit Ampicillin 100mg/l. Aus je einer Transformante wurde Plasmid-DNA isoliert. Auf diese Weise erhielt man die Plasmide pUC18aecD::dapA(MC20) und pUC18aecD::dapA(MA16).

Die Plasmide pUC18aecD::dapA(MC20) und pUC18aecD::dapA(MA16) wurden mit dem Restriktionsenzym EcoRI partiell und mit dem Enzym SalI vollständig gespalten, um das 3,0 kb große aecD::dapA(MA16) bzw aecD::dapA(MC20) tragende Fragment zu erhalten. Das Fragment wurde mit Hilfe der T4-DNA-Ligase in den geschnittenen und mit alkalischer Phosphatase behandelten Vektor pK19mobsacB (Schäfer et al., Gene (145) 69-73 (1994)) ligiert. Der Ligationsansatz wurde in den E. coli Stamm DH5 (Hanahan (1985), In: DNA cloning. A practical approach. Vol.I. IRL-Press, Oxford, Washington DC, USA) transformiert. Die Selektion der Transformanten erfolgte auf LB Medium mit Kanamycin 50mg/l. Aus je einer Transformante wurde Plasmid-DNA isoliert. Auf diese Weise erhielt man die Plasmide pK19mobsacBaecD::dapA(MC20) und pK19mobsacBaecD::dapA(MA16).

Die Plasmid DNA wurde in den E. coli Stamm S17-1 (Simon, Priefer and Pühler, Bio/Technology, (1) 784-791 (1983)) transformiert. Die Selektion der Transformanten erfolgte auf LB Medium mit Kanamycin 50mg/l. Aus je einer Transformante wurde Plasmid-DNA isoliert und überprüft. Die erhaltenen Stämme wurden S17-1/pK19mobsacBaecD::dapA(MC20) und S17-1/pK19mobsacBaecD::dapA(MA16) genannt.

### Beispiel 13

### Herstellung der C. glutamicum Stämme DSM5715aecD::dapA(MC20) und DSM5715aecD::dapA(MA16)

Die Plasmide pK19mobsacBaecD::dapA(MC20) und pK19mobsacB aecD::dapA(MA16) wurden mit der Methode der Konjugation (Schäfer et al., Journal of Bacteriology, (172) 1663-1666 (1990)) von S17-1/pK19mobsacBaecD::dapA(MC20) und S17-1/pK19mobsacBaecD::dapA(MA16) (aus Beispiel 12) in den C. glutamicum Stamm DSM5715 übertragen. Zur Selektion der Transkonjuganten wurden die Konjugationsansätze auf Hirn-Herz-Medium mit Nalidixinsäure und Kanamycin ausplattiert. Die erhaltenen Transkonjuganten wurden über Nacht in 10 ml Hirn-Herz-Medium inkubiert. Anschließend wurden Aliquots auf Sucrose-haltigen Platten (Hirn-Herz Agar mit 10% Sucrose) ausplattiert, um auf den Verlust der Sucrosesensitivität zu selektionieren. Sucrose-resistente Klone wurden isoliert und erneut auf Chloramphenicol und Kanamycin haltigen Agar-Platten (Hirn-Herz-Medium mit Kanamycin 15mg/l und Hirn-Herz-Medium mit Chloramphenicol 10 mg/l)überprüft.

Isoliert wurden Kolonien, die den folgenden Phänotyp aufwiesen:
Sucrose resistent
Kanamycin sensitiv
Chloramphenicol sensitiv.

Die Insertion des dapA Genfragmentes in das aecD-Gen wurde mit der Methode des Southern-Blots (Sambrook et al., Molecular Cloning: a Laboratory Manual (1989) Cold Spring Harbor Laboratory Press) überprüft.

### Beispiel 14

Herstellung der C. glutamicum Stämme
DSM5715aecD::dapA(MC20)/ pEC7lysE,
DSM5715aecD::dapA(MA16)/pEC7lysE,
DSM5715aecD::dapA(MC20)/pEC7, DSM5715aecD::dapA(MA16)/pEC7 und DSM5715/pEC7

Das Gen lysE liegt wie in Beispiel 3 beschrieben im Vektor pEC7 vor. Dieses Plasmid pEC7lysE sowie das Plasmid pEC7 wurde mittels Elektroporation (Haynes 1989, FEMS Microbiology Letters 61: 329-334) in die Stämme DSM5715aecD::dapA(MC20), DSM5715aecD::dapA(MA16) und DSM5715 (aus Beispiel 13) eingebracht, um so C. glutamicum DSM5715aecD::dapA(MC20)/pEC7lysE, DSM5715aecD::dapA(MA16)/pEC7lysE, DSM5715aecD::dapA(MC20)/pEC7, DSM5715aecD::dapA(MA16)/pEC7 und DSM5715/pEC7 zu erhalten. Die Selektion der Transformanten erfolgte auf Hirn-Herz-Agar mit Kanamycin 25mg/l. Aus je einer Transformante wurde Plasmid-DNA isoliert und überprüft.

Auf diese Weise erhielt man die Stämme DSM5715aecD::dapA(MC20)/pEC7lysE, DSM5715aecD::dapA(MA16)/pEC7lysE, DSM5715aecD::dapA(MC20)/pEC7, DSM5715aecD::dapA(MA16)/pEC7 und DSM5715/pEC7.

### Beispiel 15

### Herstellung von Lysin mit den in Beispiel 14 hergestellten Stämmen

Die Stämme DSM5715aecD::dapA(MC20)/pEC7lysE, DSM5715aecD::dapA(MA16)/pEC7lysE,DSMS571aecD::dapA (MC20) /pEC7, DSM5715aecD::dapA(MA16)/pEC7 und DSM5715/pEC7 wurden wie in Beispiel 8 beschrieben nach Vorkultivierung in Medium CgIII (Kase & Nakayama, Agricultural and Biological Chemistry 36 (9) 1611- 1621 (1972)) im Produktionsmedium MM kultiviert. Nach 48 Stunden Inkubation wurde die optische Dichte bei 660nm und die Konzentration an gebildetem L-Lysin bestimmt.

In Tabelle 2 ist das Ergebnis des Versuches dargestellt.

**Tabelle 2**

| Stamm | OD | Lysin-HCl g/l |
|---|---|---|
| DSM5715aecD::dapA(MC20)/pEC7lysE | 13,3 | 13,8 |
| DSM5715aecD::dapA(MA16)/pEC7lysE | 12,4 | 14, 3 |
| DSM5715/pEC7 | 13,3 | 11,5 |
| DSM5715aecD::dapA(MA16)/pEC7 | 13,3 | 12,9 |
| DSM5715aecD::dapA(MC20)/pEC7 | 14,0 | 12,8 |

### SEQUENZ PROTOKOLL

<110> Degussa-Hüls AG
   Forschungszentrum Jülich GmbH
<120> L-Lysin produzierende coryneforme Bakterien und Verfahren zur Herstellung von L-Lysin.
<130> 990058 BT
<140>
   <141>
<160> 6
<170> PatentIn Ver. 2.1
<210> 1
   <211> 795
   <212> DNA
   <213> Corynebacterium glutamicum
<220>
   <221> -35_signal
   <222> (774)..(779)
<220>
   <223> DNA stromaufwärts von dapB
<400> 1
   ctgcagcaat gagaccgagt aatttcgggg ttgaccagat acaccaatga gaacttggga 60
   acgggcttca aaaatactgg tgaagttgat gtcttcaaca atgcctgcac caggatatga 120
   tccggtatcg atacctggaa cgacaacctg atcaggatat ccagtgcctt gaatattgac 180
   gttgaggaag gaatcaccag ccatctcaac tggaagacct gacgcctgct gaattggatc 240
   agtggcccaa tcgacccacc aaccaggttg gccattaccg gcgatatcaa aaacaactcg 300
   tgtgaacgtt tcgtgctcgg caacgcggat gccagcgatc gacatatcgg agtcaccaac 360
   ttgagcctgc tgcttctgat ccatcgacgg ggaacccaac ggcggcaaag cagtggggga 420
   aggggggagt ttggtgcact ctgaaccgag tggtctctga agtggtaggc gacggggcag 480
   ctatctgaag gcgtgcgagt tgtggtgacc gggttagcgg tttcagtttc tgtcacaact 540
   ggagcaggac tagcagaggt tgtaggcgtt gagccgcttc catcacaagc acttaaaagt 600
   aaagaggcgg aaaccacaag cgccaaggaa ctactgcgga acgggcggtg aagggcaact 660
   taagtctcat atttcaaaca tagttccacc tgtgtgatta atccctagaa cggaacaaac 720
   tgatgaacaa tcgttaacaa cacagaccaa aacggtcagt taggtatgga tatcagcacc 780
   ttctgaacgg gtacg 795
<210> 2
   <211> 1815
   <212> DNA
   <213> Corynebacterium glutamicum
<220>
   <221> -35 signal
   <222> (774)..(779)
<220>
   <221> -10 signal
   <222> (798)..(803)
<220>
   <221> CDS
   <222> (851)..(1594)
<400> 2
   ctgcagcaat gagaccgagt aatttcgggg ttgaccagat acaccaatga gaacttggga 60
   acgggcttca aaaatactgg tgaagttgat gtcttcaaca atgcctgcac caggatatga 120
   tccggtatcg atacctggaa cgacaacctg atcaggatat ccagtgcctt gaatattgac 180
   gttgaggaag gaatcaccag ccatctcaac tggaagacct gacgcctgct gaattggatc 240
   agtggcccaa tcgacccacc aaccaggttg gccattaccg gcgatatcaa aaacaactcg 300
   tgtgaacgtt tcgtgctcgg caacgcggat gccagcgatc gacatatcgg agtcaccaac 360
   ttgagcctgc tgcttctgat ccatcgacgg ggaacccaac ggcggcaaag cagtggggga 420
   aggggggagt ttggtgcact ctgaaccgag tggtctctga agtggtaggc gacggggcag 480
   ctatctgaag gcgtgcgagt tgtggtgacc gggttagcgg tttcagtttc tgtcacaact 540
   ggagcaggac tagcagaggt tgtaggcgtt gagccgcttc catcacaagc acttaaaagt 600
   aaagaggcgg aaaccacaag cgccaaggaa ctactgcgga acgggcggtg aagggcaact 660
   taagtctcat atttcaaaca tagttccacc tgtgtgatta atccctagaa cggaacaaac 720
   tgatgaacaa tcgttaacaa cacagaccaa aacggtcagt taggtatgga tatcagcacc 780
   ttctgaacgg gtacgtctag actggtgggc gtttgaaaaa ctcttcgccc cacgaaaatg 840
   aaggagcata atg gga atc aag gtt ggc gtt ctc gga gcc aaa ggc cgt   889
              Met Gly Ile Lys Val Gly Val Leu Gly Ala Lys Gly Arg
                1               5                  10
   gtt ggt caa act att gtg gca gca gtc aat gag tcc gac gat ctg gag   937
   Val Gly Gln Thr Ile Val Ala Ala Val Asn Glu Ser Asp Asp Leu Glu
        15                  20                  25
   ctt gtt gca gag atc ggc gtc gac gat gat ttg agc ctt ctg gta gac   985
   Leu Val Ala Glu Ile Gly Val Asp Asp Asp Leu Ser Leu Leu Val Asp
    30                  35                  40                  45
   aac ggc gct gaa gtt gtc gtt gac ttc acc act cct aac gct gtg atg   1033
   Asn Gly Ala Glu Val Val Val Asp Phe Thr Thr Pro Asn Ala Val Met
                    50                  55                  60
   ggc aac ctg gag ttc tgc atc aac aac ggc att tct gcg gtt gtt gga   1081
   Gly Asn Leu Glu Phe Cys Ile Asn Asn Gly Ile Ser Ala Val Val Gly
                65                  70                  75
   acc acg ggc ttc gat gat gct cgt ttg gag cag gtt cgc gac tgg ctt   1129
   Thr Thr Gly Phe Asp Asp Ala Arg Leu Glu Gln Val Arg Asp Trp Leu
            80                  85                  90
   gaa gga aaa gac aat gtc ggt gtt ctg atc gca cct aac ttt gct atc   1177
   Glu Gly Lys Asp Asn Val Gly Val Leu Ile Ala Pro Asn Phe Ala Ile
        95                 100                 105
   tct gcg gtg ttg acc atg gtc ttt tcc aag cag gct gcc cgc ttc ttc   1225
   Ser Ala Val Leu Thr Met Val Phe Ser Lys Gln Ala Ala Arg Phe Phe
   110                 115                 120                 125
   gaa tca gct gaa gtt att gag ctg cac cac ccc aac aag ctg gat gca   1273
   Glu Ser Ala Glu Val Ile Glu Leu His His Pro Asn Lys Leu Asp Ala
                   130                 135                 140
   cct tca ggc acc gcg atc cac act gct cag ggc att gct gcg gca cgc    1321
   Pro Ser Gly Thr Ala Ile His Thr Ala Gln Gly Ile Ala Ala Ala Arg
               145                 150                 155
   aaa gaa gca ggc atg gac gca cag cca gat gcg acc gag cag gca ctt    1369
   Lys Glu Ala Gly Met Asp Ala Gln Pro Asp Ala Thr Glu Gln Ala Leu
           160                 165                 170
   gag ggt tcc cgt ggc gca agc gta gat gga atc ccg gtt cat gca gtc    1417
   Glu Gly Ser Arg Gly Ala Ser Val Asp Gly Ile Pro Val His Ala Val
       175                 180                 185
   cgc atg tcc ggc atg gtt gct cac gag caa gtt atc ttt ggc acc cag    1465
   Arg Met Ser Gly Met Val Ala His Glu Gln Val Ile Phe Gly Thr Gln
   190                 195                 200                 205
   ggt cag acc ttg acc atc aag cag gac tcc tat gat cgc aac tca ttt    1513
   Gly Gln Thr Leu Thr Ile Lys Gln Asp Ser Tyr Asp Arg Asn Ser Phe
                   210                 215                 220
   gca cca ggt gtc ttg gtg ggt gtg cgc aac att gca cag cac cca ggc    1561
   Ala Pro Gly Val Leu Val Gly Val Arg Asn Ile Ala Gln His Pro Gly
               225                 230                 235
   cta gtc gta gga ctt gag cat tac cta ggc ctg taaaggctca tttcagcagc 1614
   Leu Val Val Gly Leu Glu His Tyr Leu Gly Leu
           240                 245
   gggtggaatt ttttaaaagg agcgtttaaa ggctgtggcc gaacaagtta aattgagcgt 1674
   ggagttgata gcgtgcagtt cttttactcc acccgctgat gttgagtggt caactgatgt 1734
   tgagggcgcg gaagcactcg tcgagtttgc gggtcgtgcc tgctacgaaa cttttgataa 1794
   gccgaaccct cgaactgctt c                                           1815
<210> 3
   <211> 248
   <212> PRT
   <213> Corynebacterium glutamicum
<400> 3
   Met Gly Ile Lys Val Gly Val Leu Gly Ala Lys Gly Arg Val Gly Gln
    1               5                  10                  15
   Thr Ile Val Ala Ala Val Asn Glu Ser Asp Asp Leu Glu Leu Val Ala
               20                  25                  30
   Glu Ile Gly Val Asp Asp Asp Leu Ser Leu Leu Val Asp Asn Gly Ala
           35                  40                  45
   Glu Val Val Val Asp Phe Thr Thr Pro Asn Ala Val Met Gly Asn Leu
       50                  55                  60
   Glu Phe Cys Ile Asn Asn Gly Ile Ser Ala Val Val Gly Thr Thr Gly
   65                  70                  75                  80
   Phe Asp Asp Ala Arg Leu Glu Gln Val Arg Asp Trp Leu Glu Gly Lys
                   85                  90                  95
   Asp Asn Val Gly Val Leu Ile Ala Pro Asn Phe Ala Ile Ser Ala Val
               100                 105                 110
   Leu Thr Met Val Phe Ser Lys Gln Ala Ala Arg Phe Phe Glu Ser Ala
           115                 120                 125
   Glu Val Ile Glu Leu His His Pro Asn Lys Leu Asp Ala Pro Ser Gly
       130                 135                 140
   Thr Ala Ile His Thr Ala Gln Gly Ile Ala Ala Ala Arg Lys Glu Ala
   145                 150                 155                 160
   Gly Met Asp Ala Gln Pro Asp Ala Thr Glu Gln Ala Leu Glu Gly Ser
                   165                 170                 175
   Arg Gly Ala Ser Val Asp Gly Ile Pro Val His Ala Val Arg Met Ser
               180                 185                 190
   Gly Met Val Ala His Glu Gln Val Ile Phe Gly Thr Gln Gly Gln Thr
           195                 200                 205
   Leu Thr Ile Lys Gln Asp Ser Tyr Asp Arg Asn Ser Phe Ala Pro Gly
       210                 215                 220
   Val Leu Val Gly Val Arg Asn Ile Ala Gln His Pro Gly Leu Val Val
   225                 230                 235                 240
   Gly Leu Glu His Tyr Leu Gly Leu
                   245
<210> 4
   <211> 79
   <212> DNA
   <213> Corynebacterium glutamicum
<220>
   <223> dapA Wildtyp-Promotor
<400> 4
   gttaggtttt ttgcggggtt gtttaacccc caaatgaggg aagaaggtaa ccttgaactc 60
   tatgagcaca ggtttaaca                                              79
<210> 5
   <211> 79
   <212> DNA
   <213> Künstliche Sequenz
<220>
   <223> Beschreibung der künstlichen Sequenz: dapA-Promotor von C. glutamicum mit der MC20-Mutation
<220>
   <221> mutation
   <222> (45)
<400> 5
   gttaggtttt ttgcggggtt gtttaacccc caaatgaggg aagatggtaa ccttgaactc 60
   tatgagcaca ggtttaaca                                              79
<210> 6
   <211> 80
   <212> DNA
   <213> Künstliche Sequenz
<220>
   <223> Beschreibung der künstlichen Sequenz: dapA-Promotor von C. glutamicum mit der MA16-Mutation
<220>
   <221> mutation
   <222> (35)..(53)
<400> 6

### Abbildungen:

Folgende Abbildungen sind beigefügt:
- Abbildung 1: Plasmid pEC71ysE
- Abbildung 2: Plasmid pEC7dapB
- Abbildung 3: Plasmid pEC7dapBlysE

Die in den Abbildungen verwendeten Abkürzungen haben folgende Bedeutung:
- Cm:: Resistenzgen für Chloramphenicol
- dapB:: dapB-Gen von C. glutamicum
- lysE:: lysE-Gen von C. glutamicum
- pyc:: pyc-Gen von C. glutamicum
- OriE:: Plasmidkodierter Replikationsursprung E. coli
- pBL:: DNA-Fragment des Plasmids pBL1
- EcoRI:: Schnittstelle des Restriktionsenzyms EcoRI
- EcoRV:: Schnittstelle des Restriktionsenzyms EcoRV
- HincII:: Schnittstelle des Restriktionsenzyms HincII
- HindIII:: Schnittstelle des Restriktionsenzyms HindIII
- KpnI:: Schnittstelle des Restriktionsenzyms KpnI
- SalI:: Schnittstelle des Restriktionsenzyms SalI
- SmaI:: Schnittstelle des Restriktionsenzyms SmaI
- SphI:: Schnittstelle des Restriktionsenzyms SphI
- PvuII:: Schnittstelle des Restriktionsenzyms PvuII
- BamHI:: Schnittstelle des Restriktionsenzyms BamHI

## Patentansprüche

1. L-Lysin produzierende coryneforme Bakterien mit einem überexprimierten lysE-Gen, in denen zusätzlich das dapA-Gen, überexprimiert wird, wobei der Promoter des dapA-Gens die Sequenzen dargestellt in SEQ-ID-No. 5 (Mutation MC20) oder SEQ-ID-No. 6 (Mutation MA16), aufweist.

2. Coryneforme Bakterien gemäß Anspruch 1,
in denen das dapB-Gen überexprimiert wird.

3. Coryneforme Bakterien gemäß Anspruch 2,
in denen das dapB-Gen, das zusätzlich den 5'Bereich vor dem Translationsstart dieses Gens, dargestellt in der SEQ-ID-No. 1, enthält, überexprimiert wird.

4. Coryneforme Bakterien gemäß Anspruch 1,
in denen das dapB-Gen oder das lysC-Gen, überexprimiert wird.

5. Verfahren zur Herstellung von L-Lysin durch Fermentation coryneformer Bakterien,
**dadurch gekennzeichnet,**
**daß** man Bakterien gemäß den Ansprüchen 1 bis 4 einsetzt.

6. Verfahren gemäß Anspruch 5,
**dadurch gekennzeichnet,**
**daß** man einen mit einem oder mehreren Plasmivektor(en) transformierten Stamm einsetzt, und der (die) Plasmidvektor(en) die Nucleotidsequenzen für die zu überexprimierenden Gene trägt.

7. Verfahren gemäß Anspruch 5,
**dadurch gekennzeichnet,**
**daß** man einen mit einem Plasmidvektor transformierten Stamm einsetzt, und der Plasmidvektor die Nucleotidsequenzen für die Gene dapA, lysC und lysE trägt.

8. Verfahren gemäß Anspruch 5, **dadurch gekennzeichnet,**
**dass** die überexprimierten Gene in das Chromosom der eingesetzten Bakterien integriert sind.

9. Verfahren nach einem oder mehreren der vorhergehenden Ansprüche 5 bis 8,
**dadurch gekennzeichnet,**
**daß** man Bakterien der Gattung Corynebacterium glutamicum einsetzt.

10. Verfahren zur fermentativen Herstellung von L-Lysin gemäß einem oder mehreren der vorhergehenden Ansprüche 5 bis 9,
**dadurch gekennzeichnet,**
**daß** man folgende Schritte durchführt:
a) Fermentation der L-Lysin produzierenden Bakterien, gemäß den Ansprüchen 1 bis 4,
b) Anreicherung von L-Lysin im Medium oder in den Zellen der Bakterien und
c) Isolieren des L-Lysins.

## Claims

1. L-lysine-producing coryneform bacteria which have an overexpressed lysE gene and in which the dapA gene is also overexpressed, wherein the promoter of the dapA gene has the sequences depicted in SEQ ID No. 5 (MC20 mutation) or SEQ ID No. 6 (MA16 mutation).

2. Coryneform bacteria according to Claim 1, in which the dapB gene is overexpressed.

3. Coryneform bacteria according to Claim 2, in which the dapB gene which additionally comprises the 5' region upstream of the translational start of this gene, depicted in SEQ ID No. 1, is overexpressed.

4. Coryneform bacteria according to Claim 1, in which the dapB gene or the lysC gene is overexpressed.

5. Method of producing L-lysine by fermentation of coryneform bacteria, **characterized in that** bacteria according to Claims 1 to 4 are employed.

6. Method according to Claim 5, **characterized in that** a strain transformed with one or more plasmid vector(s) is employed, said plasmid vector(s) carrying the nucleotide sequences of the genes to be overexpressed.

7. Method according to Claim 5, **characterized in that** a strain transformed with a plasmid vector is employed, said plasmid vector carrying the nucleotide sequences of the dapA, lysC and lysE genes.

8. Method according to Claim 5, **characterized in that** the overexpressed genes are integrated in the chromosome of the bacteria employed.

9. Method according to one or more of the preceding Claims 5 to 8, **characterized in that** bacteria of the genus *Corynebacterium glutamicum* are employed.

10. Method of producing L-lysine by fermentation according to one or more of the preceding claims 5 to 9, **characterized in that** the following steps are carried out:
a) fermentation of the L-lysine-producing bacteria according to Claims 1 to 4;
b) accumulation of L-lysine in the medium or in the bacterial cells; and
c) recovery of the L-lysine.

## Revendications

1. Bactéries corynéformes productrices de L-lysine, comportant un gène LysE surexprimé, dans lesquelles en outre le gène dapA est surexprimé, le promoteur du gène dapA comportant les séquences représentées dans SEQ ID n° 5 (mutation MC20) ou SEQ ID n° 6 (mutation MA16).

2. Bactéries corynéformes selon la revendication 1, dans lesquelles le gène dapB est surexprimé.

3. Bactéries corynéformes selon la revendication 2, dans lesquelles le gène dapB, qui contient en outre la région 5' avant le site d'initiation de traduction de ce gène, représentée dans la séquence SEQ ID n° 1, est surexprimé.

4. Bactéries corynéformes selon la revendication 1, dans lesquelles le gène dapB ou le gène LysC est surexprimé.

5. Procédé pour la production de L-lysine par culture par fermentation de bactéries corynéformes, **caractérisé en ce qu'**on utilise des bactéries selon les revendications 1 à 4.

6. Procédé selon la revendication 5, **caractérisé en ce qu'**on utilise une souche transformée par un ou plusieurs vecteur(s) plasmidique(s), et le (les) vecteur (s) plasmidique (s) porte (nt) les séquences nucléotidiques pour les gènes à surexprimer.

7. Procédé selon la revendication 5, **caractérisé en ce qu'**on utilise une souche transformée par un vecteur plasmidique et le vecteur plasmidique porte les séquences nucléotidiques pour les gènes dapA, LysC et LysE.

8. Procédé selon la revendication 5, **caractérisé en ce que** les gènes surexprimés sont intégrés dans le chromosome des bactéries utilisées.

9. Procédé selon une ou plusieurs des revendications précédentes 5 à 8, **caractérisé en ce qu'**on utilise des bactéries appartenant au genre *Corynebacterium.*

10. Procédé pour la production de L-lysine par fermentation selon une ou plusieurs des revendications précédentes 5 à 9, **caractérisé en ce qu'**on effectue les étapes suivantes :
a) culture par fermentation des bactéries productrices de L-Lysine, selon les revendications 1 à 4,
b) concentration de la L-lysine dans le milieu ou dans les cellules des bactéries et
c) isolement de la L-lysine.
